# EUROPEAN PATENT SPECIFICATION

(11) **EP 4 349 953 B1**
(45) Date of publication and mention of the grant of the patent: **02.09.2026**
(21) Application number: 22815986.9
(22) Date of filing: 27.05.2022
(51) Int. Cl.: G01N 35/04, G01N 35/10, C12N 15/10, C12Q 1/68

(54) **SPECIMEN TREATMENT APPARATUS**
PROBENBEHANDLUNGSVORRICHTUNG
APPAREIL DE TRAITEMENT D'ÉCHANTILLON

(30) Priority: 31.05.2021 JP 2021091568
(43) Date of publication of application: 10.04.2024
(73) Proprietor: Universal Bio Research Co., Ltd., Matsudo-shi, Chiba 2702231 (JP)
(72) Inventor: TAJIMA, Hideji, Matsudo-shi, Chiba 271-0064 (JP)
(74) Representative: J A Kemp LLP
(86) International application number: PCT/JP2022/021683
(87) International publication number: WO 2022/255236

(56) References cited:
- WO-A1-2020/093824
- JP-A- 2010 127 941
- JP-A- 2015 139 398
- JP-A- 2019 113 416
- US-A1- 2006 133 965

## Description

### Technical Field

The present invention relates to a specimen processing apparatus that concurrently extracts nucleic acids from a plurality of specimens, and more specifically relates to a specimen processing apparatus in which the pitch of a plurality of holders can be converted, the plurality of holders holding specimens and/or extracted nucleic acids.

### Background Art

For detection of pathogens, such as viruses, the PCR method is used, which has become widespread with the development of genetic engineering, and which makes use of polymerase chain reaction (PCR). A specimen (saliva, nasopharyngeal swab fluid, or the like) containing a pathogen is taken with a commercially available sampling kit, a nucleic acid (DNA or RNA) is extracted from the specimen, the nucleic acid is amplified by the PCR method, and the pathogen is then detected based on the amplified nucleic acid. The world has been exposed to the new coronavirus (COVID-19) pandemic since the end of 2019. The PCR method has been used for detecting COVID-19.

Although the PCR method is performed mainly by manual handling, manual handling not only has difficulties in continuously performing a large amount of inspection processing, but also has the possibility of erroneous operations or contamination occurring in processing steps. In view of the above, an automated PCR test device that automatically performs the PCR method is proposed. For example, a fully automated sample-to-result PCR system (gene LEAD series) made by Precision System Science Co., Ltd. can be used as the automated PCR test device. This fully automated sample-to-result PCR system can perform, without manual intervention, a PCR test by extracting specimens from specimen containers that house the specimens.

In the case of performing the PCR method without using the automated PCR test device, a plurality of nucleic acids that are manually extracted from a plurality of specimens are dispensed into a plurality of wells of a PCR plate, and the PCR method is performed with the PCR plate housed in a thermal cycler. A 96-well PCR plate is used for the PCR plate, for example. By using the 96-well PCR plate, it is possible to simultaneously amplify nucleic acids extracted from 96 specimens at maximum. Such a PCR plate is described in Figure 1b of Patent Literature 1, for example, and the thermal cycler is described in paragraph [5] of Patent Literature 1. US 2006/133965 A1 discloses a monitoring function-equipped dispensing system and a method of monitoring a dispensing device. JP 2019 113416 A discloses a fully automatic electrophoretic processing device and method thereof.

### Citation List

### Patent Literature

Patent Literature 1 : International Publication No. WO 2002/081087

### Summary of Invention

### Technical Problem

By using the above-described fully automated sample-to-result PCR system, extraction of nucleic acids from a plurality of specimens, amplification of the nucleic acids, and detection of the nucleic acids can be concurrently and continuously performed. However, this fully automated sample-to-result PCR system does not allow use of a conventional PCR plate or a thermal cycler.

In view of the above, it is an object of the present invention to provide a specimen processing apparatus that concurrently extracts nucleic acids from a plurality of specimens, and that collectively moves the extracted nucleic acids to a container, such as a PCR plate. Solution to Problem

According to the present invention, there is provided a specimen process apparatus according to appended claim 1. Preferred embodiments are defined in the dependent claims.

### Advantageous Effect of Invention

The specimen processing apparatus of the present invention can concurrently extract nucleic acids from a plurality of specimens, and can collectively move the extracted nucleic acids to a container, such as a PCR plate.

### Brief Description of Drawings

Figure 1 is a perspective view showing a specimen processing apparatus according to one embodiment of the present invention.
Figure 2 is a top plan view showing the specimen processing apparatus that includes a pitch conversion mechanism set to a larger pitch.
Figure 3 is a top plan view showing the specimen processing apparatus that includes the pitch conversion mechanism set to a smaller pitch.
Figure 4 is a perspective view showing the pitch conversion mechanism set to the smaller pitch.
Figure 5 is a perspective view showing the pitch conversion mechanism set to the larger pitch.
Figure 6 is a top plan view showing an arrangement in which a second nozzle unit is at a position above the pitch conversion mechanism set to the smaller pitch.
Figure 7 is a top plan view showing an arrangement in which a first nozzle unit is at a position above the pitch conversion mechanism set to the larger pitch.
Figure 8 is a top plan view showing an arrangement in which the first nozzle unit is at a position above the pitch conversion mechanism set to the larger pitch.

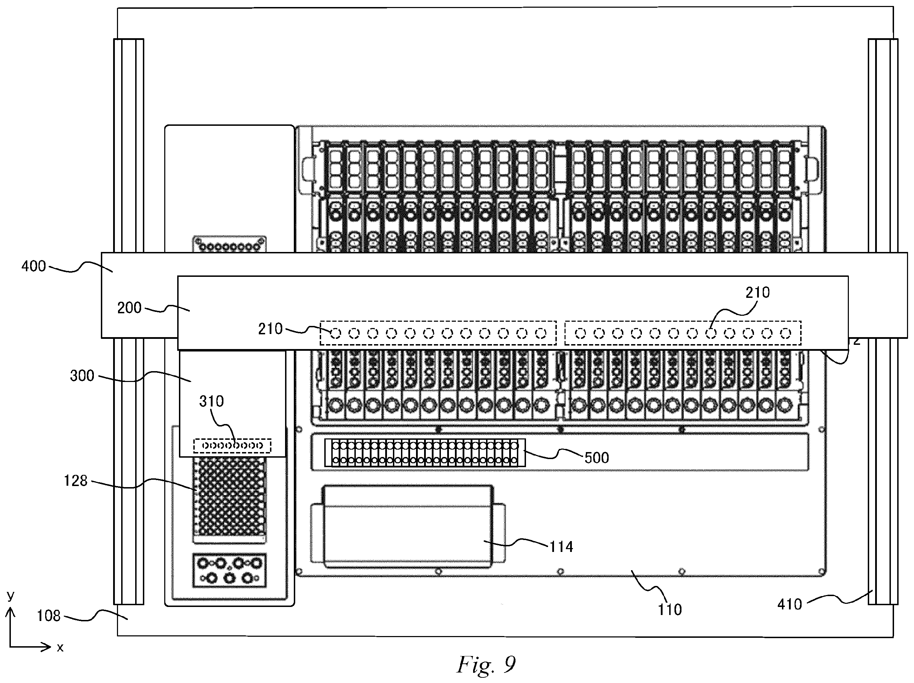

Figure 9 is a top plan view showing an arrangement in which the second nozzle unit is at a position above a PCR plate.

### Description of Embodiments

An embodiment relating to a specimen processing apparatus of the present invention will be described with reference to drawings. In the respective drawings, identical components are given the same reference symbols, and the description of such components will be omitted when appropriate. In the respective drawings, relative sizes and/or arrangement of respective components are accurately illustrated in principle. However, the present invention is not limited to such relative sizes and/or arrangement of the respective components. In the embodiment of the present invention, a specimen is a living-body-related substance, and a body fluid taken from a living body, or any dissolvable tissue may be preferably used. Note that the x direction and the y direction in the embodiment respectively correspond to the first direction and the second direction in Claims.

### [Basic configuration of specimen processing apparatus]

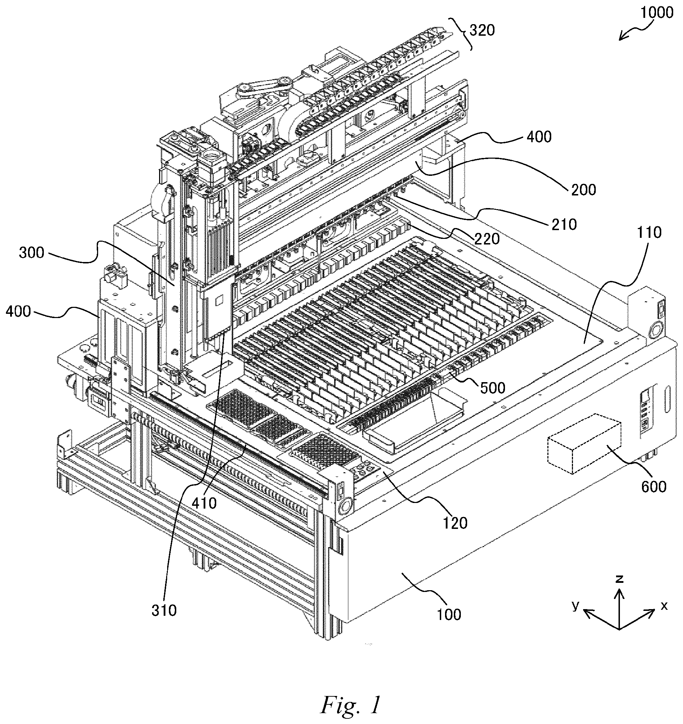

The basic configuration of a specimen processing apparatus 1000 according to an embodiment of the present invention will be described with reference to Figure 1. As shown in Figure 1, the specimen processing apparatus 1000 includes a base 100, a first nozzle unit 200, a second nozzle unit 300, a main moving mechanism 400, a pitch conversion mechanism 500, and a control unit (computer) 600. The base 100 supports respective components. The first nozzle unit 200 is provided for performing processing (extraction of nucleic acids or the like) on a plurality of specimens. The second nozzle unit 300 is provided for moving the plurality of specimens and/or a plurality of nucleic acids. The main moving mechanism 400 supports the first nozzle unit 200 and the second nozzle unit 300, and moves the first nozzle unit 200 and the second nozzle unit 300 in the y direction. The control unit 600 controls the operations of the respective components.

### [Configuration of specimen processing apparatus as viewed from above]

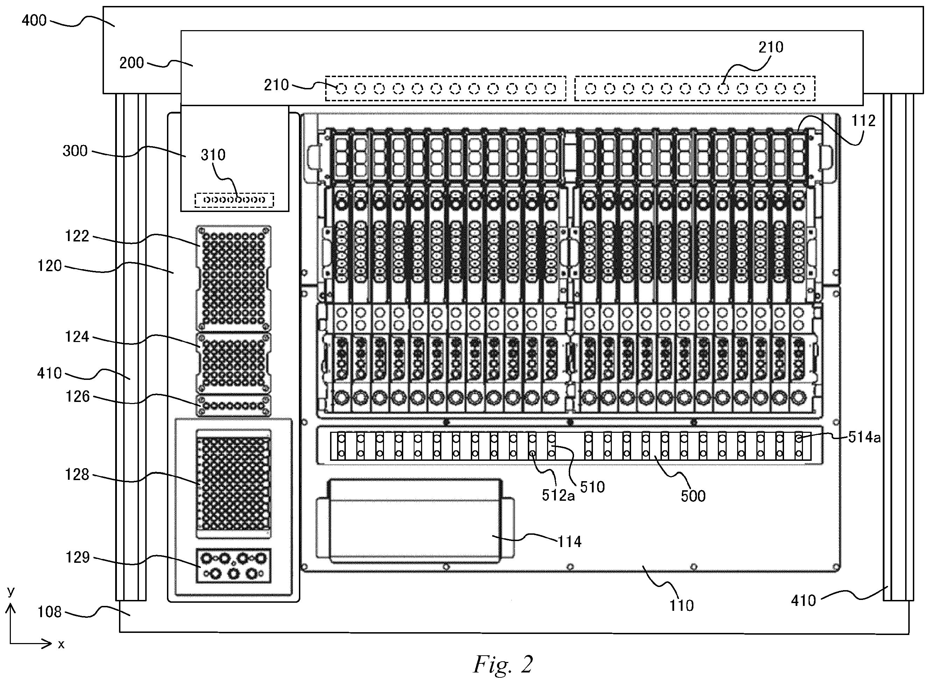

As shown in Figure 2, which is a top plan view, an upper surface 108 of the specimen processing apparatus 1000 includes a first stage 110 and a second stage 120. Processing, such as extraction of specimens, is performed on the first stage 110. Instruments, reagent, and the like used for performing processing on specimens are held on the second stage 120. The first stage 110 is provided with a plurality of processing lanes 112 and an opening 114 of a dispensing tip disposal unit. The plurality of processing lanes 112 extend in the y direction in parallel. A plurality of extraction cartridges, a plurality of dispensing tips, piercing tips, and the like are arranged in one row on each processing lane 112. Each extraction cartridge has a plurality of wells arranged in one row.

The pitch conversion mechanism 500 including a plurality of container holders 510 is disposed on one end side of the plurality of processing lanes 112. As will be described later, the pitch conversion mechanism 500 can convert the pitch of the plurality of container holders 510 in the x direction between a larger pitch (22 mm pitch, for example) and a smaller pitch (9 mm pitch, for example). It is sufficient that the larger pitch be relatively larger than the smaller pitch.

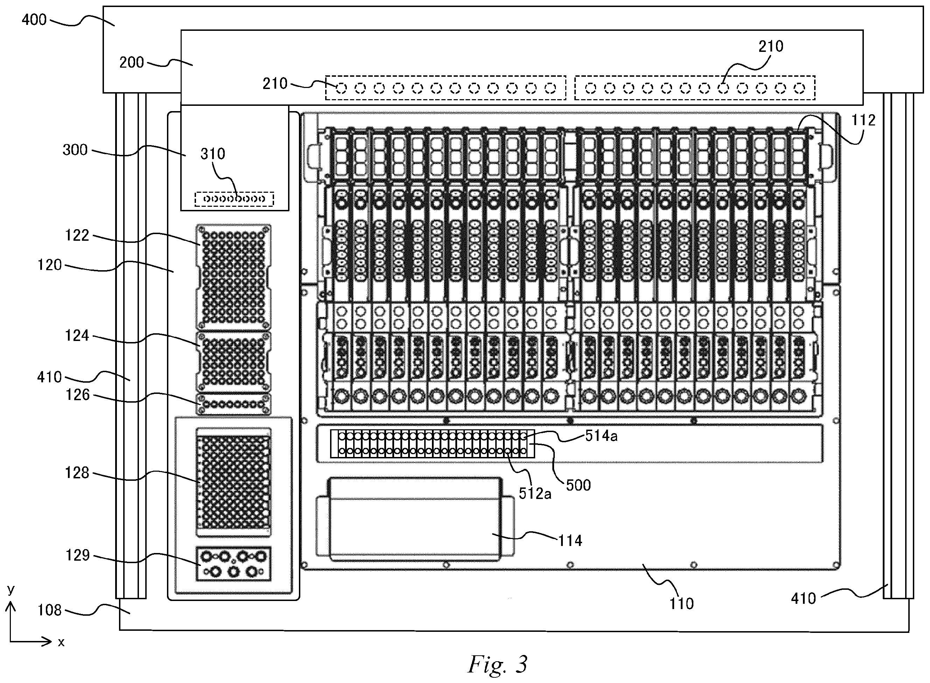

In Figure 2, the pitch conversion mechanism 500 is set to the larger pitch and hence, each container holder 510 and each of the plurality of processing lanes 112 are arranged in one row along the y direction. That is, each container holder 510 is aligned with each of the plurality of processing lanes 112 along the y direction. In Figure 3, the pitch conversion mechanism 500 is set to the smaller pitch and hence, each container holder 510 is not aligned with each of the plurality of processing lanes 112 in the y direction as a whole.

As shown in Figure 2 and Figure 3, the second stage 120 is provided with dispensing tip housing units 122, 124 for the second nozzle unit 300, a specimen container housing unit (second container housing unit) 126, a PCR plate 128 (second container housing unit), and a PCR reagent storage unit 129. The specimen container housing unit 126 is configured to house a plurality of second specimen containers in one row along the x direction. A cooling mechanism is provided below the PCR plate 128 and/or the PCR reagent storage unit 129. It is preferable that the PCR plate 128 have eight wells arranged in each row along the x direction, and have twelve wells (second nucleic acid containers) arranged in each column along the y direction.

As shown in Figure 2 and Figure 3, which are top plan views, the main moving mechanism 400 is movable in the y direction along a pair of rails 410 provided on the upper surface 108. The control unit 600 controls the movement of the main moving mechanism 400 by driving a moving motor not shown in the drawing. The first nozzle unit 200 is moved in the y direction over the first stage 110 as an integral body with the main moving mechanism 400. The first nozzle unit 200 includes a plurality of first dispensing nozzles 210 arranged in one row along the x direction. Each first dispensing nozzle 210 is movable over each processing lane 112. The first nozzle unit 200 includes a first dispensing nozzle motor that allows the plurality of first dispensing nozzles 210 to perform suction and discharge. The first nozzle unit 200 includes a magnetic force mechanism 220 (Figure 1) that exerts a magnetic force on dispensing tips, with the dispensing tips mounted at the lower end portions of the respective first dispensing nozzles 210. The magnetic force mechanism 220 includes a magnetic force mechanism motor that allows the magnetic force mechanism 220 to advance and retract in the y direction. The control unit 600 controls the operations of the respective motors.

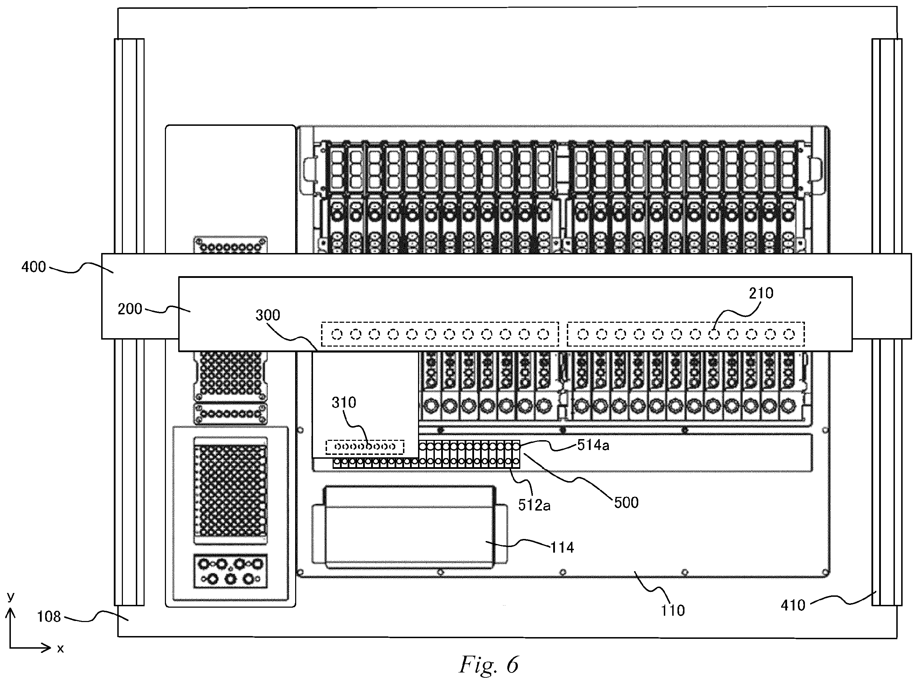

The second nozzle unit 300 is integrally formed with the main moving mechanism 400 and/or the first nozzle unit 200. The second nozzle unit 300 is moved in the y direction over the first stage 110 and the second stage 120. The second nozzle unit 300 includes a plurality of second dispensing nozzles 310 arranged in one row along the x direction. The respective second dispensing nozzles 310 are movable over the second stage 120 in the state shown in Figure 2. The respective second dispensing nozzles 310 are movable over the first stage 110 in the state shown in Figure 6.

The second nozzle unit 300 includes a second dispensing nozzle motor that allows the plurality of second dispensing nozzles 310 to perform suction and discharge. The control unit 600 controls the operations of the respective motors. The second nozzle unit 300 includes a sub-moving mechanism 320 (Figure 1) that moves the second nozzle unit 300 in the x direction with respect to the main moving mechanism 400 and/or the first nozzle unit 200. The control unit 600 controls the operation of the sub-moving mechanism 320. The sub-moving mechanism 320 is provided to the main moving mechanism 400.

### [Pitch conversion mechanism]

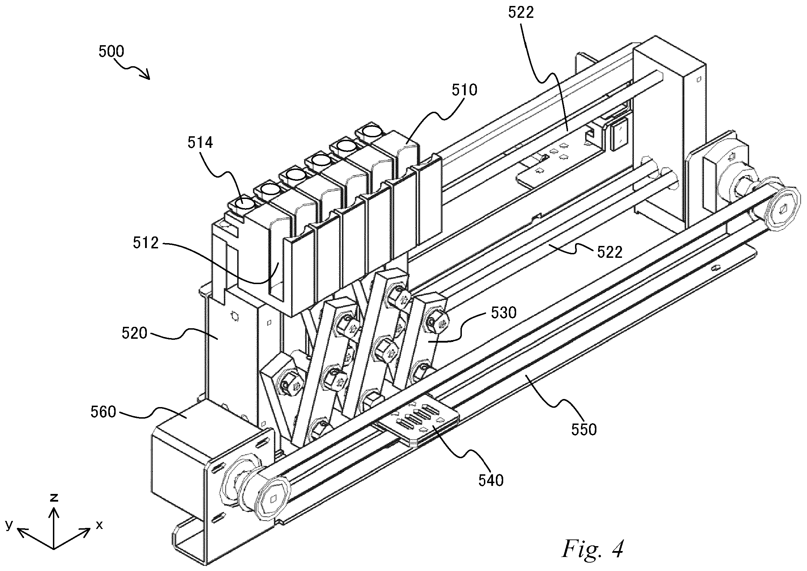

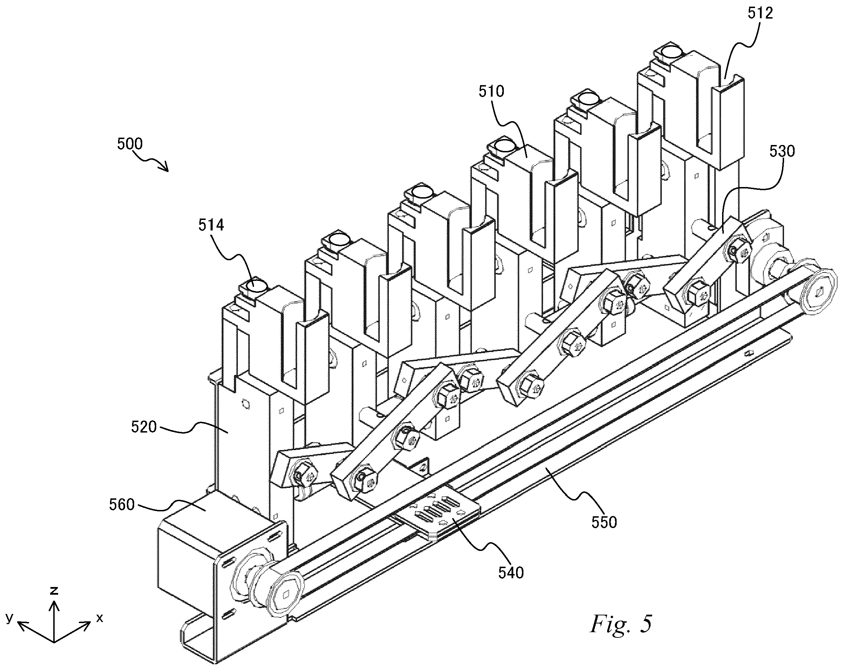

The pitch conversion mechanism 500 will be described with reference to Figure 4 and Figure 5. In Figure 4 and Figure 5, portions of the pitch conversion mechanism 500 that are repeated in the x direction are illustrated in a partially omitted manner. The pitch conversion mechanism 500 set to a smaller pitch is shown in Figure 3 and Figure 4, and the pitch conversion mechanism 500 set to a larger pitch is shown in Figure 2 and Figure 5.

As shown in Figure 4 and Figure 5, the pitch conversion mechanism 500 includes the plurality of container holders 510, nucleic acid container holding spaces 512, specimen container holding spaces 514, a plurality of holder support parts 520, one or a plurality of guide rod (guide part) 522, a link mechanism 530, a slide piece 540, a drive belt 550, and a drive belt motor 560. The nucleic acid container holding space 512 is provided to each of the plurality of container holders 510. The specimen container holding space 514 is provided to each of the plurality of container holders 510. The plurality of holder support parts 520 support the plurality of container holders 510 such that each of the plurality of container holders 510 is movable in the x direction. The one or the plurality of guide rod 522 guides the plurality of holder support parts 520 in the x direction. The link mechanism 530 is connected to each of the plurality of holder support parts 520. The slide piece 540 is connected to the link mechanism 530 to cause the link mechanism 530 to slide between the smaller pitch and the larger pitch. The drive belt 550 causes the slide piece 540 to slide. The drive belt motor 560 drives the drive belt 550. A slide piece drive mechanism includes the drive belt 550 and the drive belt motor 560. The control unit 600 controls the operation of the slide piece drive mechanism (the drive belt motor 560).

A nucleic acid container 512a (Figure 2) is held in each nucleic acid container holding space 512, and houses a nucleic acid extracted from a specimen. A specimen container 514a (Figure 2) is held in each specimen container holding space 514, and houses a specimen transferred by the second nozzle 310 from the specimen container storage unit 126 on the second stage 120. The plurality of specimen container holding spaces 514 are disposed at a position closer to the plurality of processing lanes 112 than the plurality of nucleic acid container holding spaces 512 in the y direction. In other words, the plurality of container holders 510 house the plurality of specimen containers 514a at a position closer to the plurality of processing lanes 112 than the plurality of nucleic acid containers 512a in the y direction.

### [Operation of specimen processing apparatus]

The operation of the specimen processing apparatus will be described. First, in a first step, the second nozzle unit 300 transfers a plurality of specimens from the second stage 120 to the first stage 110. In the first step, the control unit 600 moves the second nozzle unit 300 in the y direction from the state shown in Figure 3 by driving the main moving mechanism 400, and then stops the movement of the second nozzle unit 300 when the plurality of second nozzles 310 reach a position above a plurality of second specimen containers in the specimen container storage unit 126. In the state in which the plurality of second nozzles 310 are at the position above the plurality of second specimen containers, the plurality of second nozzles 310 are moved downward (in the z direction) in order to suction a plurality of specimens into a plurality of dispensing tips, which are mounted on the plurality of second nozzles 310, from the plurality of specimen containers in the specimen container storage unit 126.

Next, in the state in which the plurality of specimens are suctioned into the plurality of dispensing tips, the control unit 600 moves the second nozzle unit 300 to the state shown in Figure 6. When the second nozzle unit 300 is in the state shown in Figure 6, the plurality of second nozzles 310 are at a position above the plurality of specimen containers 514a. In the state shown in Figure 6 in which the plurality of second nozzles 310 are at the position above the plurality of second specimen containers 514a, the plurality of second nozzles 310 are moved downward to discharge the plurality of specimens into the plurality of second specimen containers 514a.

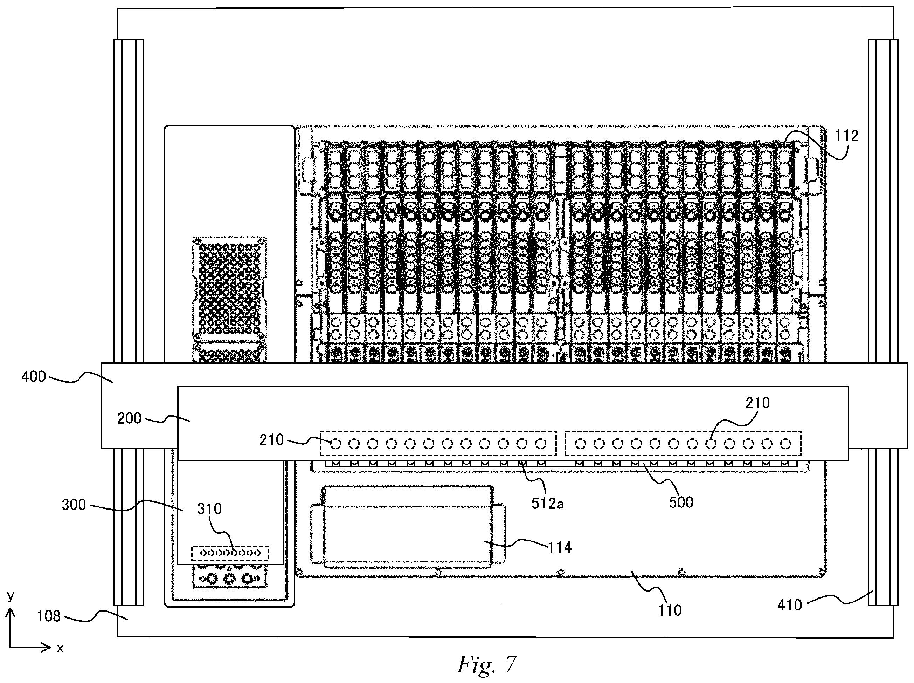

In a second step, the first nozzle unit 200 extracts nucleic acids from the plurality of specimens housed in the plurality of second specimen containers 514a on the first stage 110. After the plurality of specimens are transferred to the plurality of specimen containers 514a in the first step, in the second step, the first nozzle unit 200 and the second nozzle unit 300 are moved to the state shown in Figure 2, and the pitch conversion mechanism 500 is moved to the larger pitch state shown in Figure 2. Then, the first nozzle unit 200 is moved along the y direction to the position shown in Figure 7. When the first nozzle unit 200 is in the position shown in Figure 7, the pitch conversion mechanism 500 is set to the larger pitch, and the plurality of first nozzles 210 of the first nozzle unit 200 are at a position above the plurality of first specimen containers 514a of the pitch conversion mechanism 500. In the state in which the plurality of first nozzles 210 are at the position above the plurality of first specimen containers 514a, the plurality of first nozzles 210 are moved downward in order to suction the plurality of specimens in the plurality of first specimen containers 514a of the pitch conversion mechanism 500 into a plurality of dispensing tips mounted on the plurality of first nozzles 210.

The plurality of specimens are transferred to the plurality of processing lanes 112 in a state of being suctioned into the plurality of dispensing tips mounted on the plurality of first nozzles 210. The plurality of specimens transferred to the plurality of processing lanes 112 are subject to nucleic acid extraction processing by using the plurality of processing lanes 112 and the plurality of dispensing tips mounted on the plurality of first nozzles 210. Nucleic acid extraction is performed by a technique described in International Publication No. WO94/44671 filed by the inventors of the present invention, for example, that is, by using magnetic particles that bind to a target nucleic acid and a magnetic force mechanism (movable magnet) that adsorbs magnetic particles via a dispensing tip.

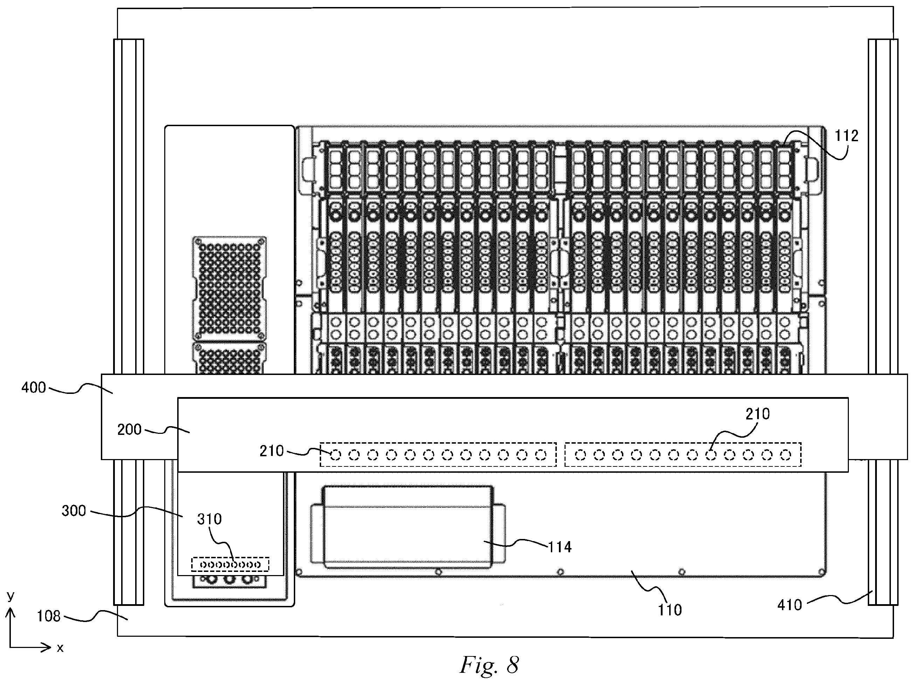

In a third step, the second nozzle unit 300 transfers the plurality of nucleic acids extracted on the first stage 110 to the PCR plate 128 on the second stage 120. First, after the extraction of nucleic acids on the plurality of processing lanes 112 is completed in the second step, the first nozzle unit 200 is moved to the position shown in Figure 8 with the extracted nucleic acids held in the plurality of dispensing tips mounted on the plurality of first nozzles 210.

When the first nozzle unit 200 is in the position shown in Figure 8, the pitch conversion mechanism 500 is set to the larger pitch, and the plurality of first nozzles 210 of the first nozzle unit 200 are at the position above the plurality of first nucleic acid containers 512a of the pitch conversion mechanism 500. In the state in which the plurality of first nozzles 210 are at the position above the plurality of first nucleic acid containers 512a, the control unit 600 moves the plurality of first nozzles 210 downward in order to discharge a nucleic acid solution in the plurality of dispensing tips, mounted on the plurality of first nozzles 210, into the plurality of first nucleic acid containers 512a of the pitch conversion mechanism 500.

After the nucleic acid solution is discharged into the first nucleic acid containers 512a, the control unit 600 sets the pitch conversion mechanism 500 to the smaller pitch, and transfers the nucleic acid solution in the plurality of first nucleic acid containers 512a into a plurality of wells of the PCR plate 128 by using the plurality of second nozzles 310 of the second nozzle unit 300, the plurality of wells being arranged in one row along the x direction. After the second nozzle unit 300 transfers the nucleic acids to the PCR plate 128 in the third step, the operator moves the PCR plate 128 to a normal thermal cycler in order to perform PCR.

As a modification of the present embodiment, a pitch conversion mechanism moving mechanism may be provided that moves the pitch conversion mechanism 500 to the second stage 120 along the x direction.

### Reference Signs List

- 1000: specimen processing apparatus
- 100: base
- 200: first nozzle unit
- 210: first nozzle
- 300: second nozzle unit
- 310: second nozzle
- 400: main moving mechanism
- 500: pitch conversion mechanism
- 510: container holder
- 600: control unit

## Claims

1. A specimen processing apparatus (1000) extracting nucleic acids from each of a plurality of specimens, the specimen processing apparatus comprising:
a plurality of container holders (510) configured to hold a plurality of first specimen containers (514a) and/or a plurality of first nucleic acid containers (512a) in one row along a first direction, the plurality of first specimen containers housing the plurality of specimens along the first direction, the plurality of first nucleic acid containers housing the nucleic acids extracted along the first direction;
a pitch conversion mechanism (500) configured to convert a pitch of the plurality of container holders along the first direction between a smaller pitch and a larger pitch;
a plurality of processing lanes (112) extending in a second direction in parallel in order to concurrently extract the nucleic acids from the plurality of specimens;
a first nozzle unit (200) including a plurality of first nozzles (210) arranged in one row along the first direction at the larger pitch, the first nozzle unit being configured to move over the plurality of processing lanes along the second direction;
a second container housing unit (126) configured to house a plurality of second specimen containers (514a) and/or a plurality of second nucleic acid containers (512a), the plurality of second specimen containers being arranged in one row along the first direction at the smaller pitch, the plurality of second nucleic acid containers being arranged in one row along the first direction at the smaller pitch;
a second nozzle unit (300) including a plurality of second nozzles (310) arranged in one row along the first direction at the smaller pitch, the second nozzle unit being configured to move between a region above the plurality of container holders set to the smaller pitch and a region above the second container housing unit; and
a control unit (600) configured to control an operation of the pitch conversion mechanism (500), an operation of the first nozzle unit (200), and an operation of the second nozzle unit (300).

2. The specimen processing apparatus (1000) according to claim 1, comprising a main moving mechanism (400) configured to move the first nozzle unit (200) in the second direction.

3. The specimen processing apparatus (1000) according to claim 1, wherein the main moving mechanism (400) is configured to move the second nozzle unit (300) in the second direction.

4. The specimen processing apparatus (1000) according to claim 1, wherein the main moving mechanism (400) is configured to move the first nozzle unit (200) and the second nozzle unit (300) as an integral body.

5. The specimen processing apparatus (1000) according to claim 1, comprising a sub-moving mechanism (320) configured to move the second nozzle unit (300) in the first direction.

6. The specimen processing apparatus (1000) according to claim 1, wherein the second container housing unit (126) that houses the plurality of second nucleic acid containers (512a) is a PCR plate (128) having a plurality of wells.

7. The specimen processing apparatus (1000) according to claim 1, comprising a pitch conversion mechanism moving mechanism configured to move the pitch conversion mechanism (500) in the first direction.

8. The specimen processing apparatus (1000) according to claim 1, wherein the plurality of container holders (510), the pitch conversion mechanism (500), and the plurality of processing lanes (112) are disposed on a first stage (110).

9. The specimen processing apparatus (1000) according to claim 8, wherein the first nozzle unit (200) is configured to move over the first stage (110).

10. The specimen processing apparatus (1000) according to claim 8, wherein the first stage (110) is provided with a dispensing tip disposal unit configured to dispose of dispensing tips mounted on the plurality of first nozzles (210) and/or dispensing tips mounted on the plurality of second nozzles (310).

11. The specimen processing apparatus (1000) according to claim 1, wherein the second container housing unit (126) is disposed on a second stage (120).

12. The specimen processing apparatus (1000) according to claim 11, comprising a cooling mechanism configured to cool the second container housing unit (126).

13. The specimen processing apparatus (1000) according to claim 11, wherein the second nozzle unit (300) is configured to move over at least the second stage (120).

14. The specimen processing apparatus (1000) according to claim 1, wherein the plurality of container holders (510) house the plurality of specimen containers (514a) at a position closer to the plurality of processing lanes (112) than the plurality of nucleic acid containers (512a) in the second direction.

15. The specimen processing apparatus (1000) according to claim **1,** wherein the pitch conversion mechanism (500) includes a plurality of holder support parts (520), a link mechanism (530), a slide piece (540), and a slide piece drive mechanism (550, 560), the plurality of holder support parts being configured to support the plurality of container holders (510) such that each of the plurality of container holders is movable in the first direction, the link mechanism being connected to the plurality of holder support parts, the slide piece being configured to cause the link mechanism to slide between the smaller pitch and the larger pitch, the slide piece drive mechanism being configured to drive the slide piece.

## Patentansprüche

1. Probenverarbeitungsvorrichtung (1000), die Nukleinsäuren aus jeder aus einer Vielzahl von Proben extrahiert, wobei die Probenverarbeitungsvorrichtung Folgendes umfasst:
eine Vielzahl von Behälterhalterungen (510), die konfiguriert sind, um eine Vielzahl von ersten Probenbehältern (514a) und/oder eine Vielzahl von ersten Nukleinsäurebehältern (512a) in einer Reihe entlang einer ersten Richtung zu halten, wobei die Vielzahl von ersten Probenbehältern die Vielzahl von Proben entlang der ersten Richtung unterbringen, wobei die Vielzahl von ersten Nukleinsäurebehältern die Nukleinsäuren unterbringen, die entlang der ersten Richtung extrahiert werden;
einen Abstandsumwandlungsmechanismus (500), der konfiguriert ist, um einen Abstand der Vielzahl von Behälterhalterungen entlang der ersten Richtung zwischen einem kleineren Abstand und einem größeren Abstand umzuwandeln;
eine Vielzahl von Verarbeitungsspuren (112), die sich in eine zweite Richtung parallel erstrecken, um die Nukleinsäuren gleichzeitig aus der Vielzahl von Proben zu extrahieren;
eine erste Düseneinheit (200) einschließlich einer Vielzahl von ersten Düsen (210), die in einer Reihe entlang der ersten Richtung in dem größeren Abstand angeordnet sind, wobei die erste Düseneinheit konfiguriert ist, um sich über die Vielzahl von Verarbeitungsspuren entlang der zweiten Richtung zu bewegen;
eine zweite Behältergehäuseeinheit (126), die konfiguriert ist, um eine Vielzahl von zweiten Probenbehältern (514a) und/oder eine Vielzahl von zweiten Nukleinsäurebehältern (512a) unterzubringen, wobei die Vielzahl von zweiten Probenbehältern in einer Reihe entlang der ersten Richtung in dem kleineren Abstand angeordnet ist, wobei die Vielzahl von zweiten Nukleinsäurebehältern in einer Reihe entlang der ersten Richtung in dem kleineren Abstand angeordnet ist;
eine zweite Düseneinheit (300) einschließlich einer Vielzahl von zweiten Düsen (310), die in einer Reihe entlang der ersten Richtung in dem kleineren Abstand angeordnet ist, wobei die zweite Düseneinheit konfiguriert ist, um sich zwischen einem Bereich über der Vielzahl von Behälterhalterungen, die in dem kleineren Abstand eingestellt sind, und einem Bereich über der zweiten Behältergehäuseeinheit zu bewegen; und
eine Steuerungseinheit (600), die konfiguriert ist, um einen Betrieb des Abstandsumwandlungsmechanismus (500), einen Betrieb der ersten Düseneinheit (200) und einen Betrieb der zweiten Düseneinheit (300) zu steuern.

2. Probenverarbeitungsvorrichtung (1000) nach Anspruch 1, umfassend einen Hauptbewegungsmechanismus (400), der konfiguriert ist, um die erste Düseneinheit (200) in die zweite Richtung zu bewegen.

3. Probenverarbeitungsvorrichtung (1000) nach Anspruch 1, wobei der Hauptbewegungsmechanismus (400) konfiguriert ist, um die zweite Düseneinheit (300) in die zweite Richtung zu bewegen.

4. Probenverarbeitungsvorrichtung (1000) nach Anspruch 1, wobei der Hauptbewegungsmechanismus (400) konfiguriert ist, um die erste Düseneinheit (200) und die zweite Düseneinheit (300) als einen einstückigen Körper zu bewegen.

5. Probenverarbeitungsvorrichtung (1000) nach Anspruch 1, umfassend einen Unterbewegungsmechanismus (320), der konfiguriert ist, um die zweite Düseneinheit (300) in die erste Richtung zu bewegen.

6. Probenverarbeitungsvorrichtung (1000) nach Anspruch 1, wobei die zweite Behältergehäuseeinheit (126), die die Vielzahl von zweiten Nukleinsäurebehältern (512a) unterbringt, eine PCR-Platte (128) mit einer Vielzahl von Kavitäten ist.

7. Probenverarbeitungsvorrichtung (1000) nach Anspruch 1, umfassend einen Abstandsumwandlungsmechanismus-Bewegungsmechanismus, der konfiguriert ist, um den Abstandsumwandlungsmechanismus (500) in die erste Richtung zu bewegen.

8. Probenverarbeitungsvorrichtung (1000) nach Anspruch 1, wobei die Vielzahl von Behälterhalterungen (510), der Abstandsumwandlungsmechanismus (500) und die Vielzahl von Verarbeitungsspuren (112) auf einer ersten Plattform (110) angeordnet sind.

9. Probenverarbeitungsvorrichtung (1000) nach Anspruch 8, wobei die erste Düseneinheit (200) konfiguriert ist, um sich über die erste Plattform (110) zu bewegen.

10. Probenverarbeitungsvorrichtung (1000) nach Anspruch 8, wobei die erste Plattform (110) mit einer Abgabespitze-Entsorgungseinheit bereitgestellt ist, die konfiguriert ist, um Abgabespitzen, die an der Vielzahl von ersten Düsen (210) angebracht sind, und/oder Abgabespitzen, die an der Vielzahl von zweiten Düsen (310) angebracht sind, zu entsorgen.

11. Probenverarbeitungsvorrichtung (1000) nach Anspruch 1, wobei die zweite Behältergehäuseeinheit (126) auf einer zweiten Plattform (120) angeordnet ist.

12. Probenverarbeitungsvorrichtung (1000) nach Anspruch 11, umfassend einen Kühlmechanismus, der konfiguriert ist, um die zweite Behältergehäuseeinheit (126) zu kühlen.

13. Probenverarbeitungsvorrichtung (1000) nach Anspruch 11, wobei die zweite Düseneinheit (300) konfiguriert ist, um sich über mindestens die zweite Plattform (120) zu bewegen.

14. Probenverarbeitungsvorrichtung (1000) nach Anspruch 1, wobei die Vielzahl von Behälterhalterungen (510) die Vielzahl von Probenbehältern (514a) an einer Position unterbringen, die näher an der Vielzahl von Verarbeitungsspuren (112) ist als die Vielzahl von Nukleinsäurebehältern (512a) in der zweiten Richtung.

15. Probenverarbeitungsvorrichtung (1000) nach Anspruch 1, wobei der Abstandsumwandlungsmechanismus (500) eine Vielzahl von Halterungsstützteilen (520), einen Verbindungsmechanismus (530), ein Gleitstück (540) und einen Gleitstück-Antriebsmechanismus (550, 560) beinhaltet, wobei die Vielzahl von Halterungsstützteilen konfiguriert sind, um die Vielzahl von Behälterhalterungen (510) zu stützen, sodass jede aus der Vielzahl von Behälterhalterungen in die erste Richtung bewegt werden kann, wobei der Verbindungsmechanismus mit der Vielzahl von Halterungsstützteilen verbunden ist, wobei das Gleitstück konfiguriert ist, um zu verursachen, dass der Verbindungsmechanismus zwischen dem kleineren Abstand und dem größeren Abstand gleitet, wobei der Gleitstück-Antriebsmechanismus konfiguriert ist, um das Gleitstück anzutreiben.

## Revendications

1. Appareil de traitement d'échantillon (1000) extrayant des acides nucléiques à partir de chacun d'une pluralité d'échantillons, l'appareil de traitement d'échantillon comprenant :
une pluralité de porte-contenants (510) configurés pour porter une pluralité de premiers contenants d'échantillon (514a) et/ou une pluralité de premiers contenants d'acide nucléique (512a) en une rangée le long d'une première direction, la pluralité de premiers contenants d'échantillon logeant la pluralité d'échantillons le long de la première direction, la pluralité de premiers contenants d'acide nucléique logeant les acides nucléiques extraits le long de la première direction ;
un mécanisme de conversion d'écartement (500) configuré pour convertir un écartement de la pluralité de porte-contenants le long de la première direction entre un écartement plus petit et un écartement plus grand ;
une pluralité de couloirs de traitement (112) s'étendant dans une deuxième direction en parallèle afin d'extraire simultanément les acides nucléiques à partir de la pluralité d'échantillons ;
une unité première buse (200) comportant une pluralité de premières buses (210) agencées en une rangée le long de la première direction à l'écartement plus grand, l'unité première buse étant configurée pour se déplacer sur la pluralité de couloirs de traitement le long de la deuxième direction ;
une unité de logement de deuxièmes contenants (126) configurée pour loger une pluralité de deuxièmes contenants d'échantillon (514a) et/ou une pluralité de deuxièmes contenants d'acide nucléique (512a), la pluralité de deuxièmes contenants d'échantillon étant agencés en une rangée le long de la première direction à l'écartement plus petit, la pluralité de deuxièmes contenants d'acide nucléique étant agencés en une rangée le long de la première direction à l'écartement plus petit ;
une unité deuxième buse (300) comportant une pluralité de deuxièmes buses (310) agencées en une rangée le long de la première direction à l'écartement plus petit, l'unité deuxième buse étant configurée pour se déplacer entre une région au-dessus de la pluralité de porte-contenants réglés sur l'écartement plus petit et une région au-dessus de l'unité de logement de deuxièmes contenants ; et
une unité de commande (600) configurée pour commander un fonctionnement du mécanisme de conversion d'écartement (500), un fonctionnement de l'unité première buse (200), et un fonctionnement de l'unité deuxième buse (300).

2. Appareil de traitement d'échantillon (1000) selon la revendication 1, comprenant un mécanisme de déplacement principal (400) configuré pour déplacer l'unité première buse (200) dans la deuxième direction.

3. Appareil de traitement d'échantillon (1000) selon la revendication 1, dans lequel le mécanisme de déplacement principal (400) est configuré pour déplacer l'unité deuxième buse (300) dans la deuxième direction.

4. Appareil de traitement d'échantillon (1000) selon la revendication 1, dans lequel le mécanisme de déplacement principal (400) est configuré pour déplacer l'unité première buse (200) et l'unité deuxième buse (300) comme un corps d'un seul tenant.

5. Appareil de traitement d'échantillon (1000) selon la revendication 1, comprenant un mécanisme de déplacement secondaire (320) configuré pour déplacer l'unité deuxième buse (300) dans la première direction.

6. Appareil de traitement d'échantillon (1000) selon la revendication, dans lequel l'unité de logement de deuxièmes contenants (126) qui loge la pluralité de deuxièmes contenants d'acide nucléique (512a) est une plaque PCR (128) ayant une pluralité de puits.

7. Appareil de traitement d'échantillon (1000) selon la revendication 1, comprenant un mécanisme de déplacement de mécanisme de conversion d'écartement configuré pour déplacer le mécanisme de conversion d'écartement (500) dans la première direction.

8. Appareil de traitement d'échantillon (1000) selon la revendication 1, dans lequel la pluralité de porte-contenants (510), le mécanisme de conversion d'écartement (500), et la pluralité de couloirs de traitement (112) sont disposés sur une première platine (110).

9. Appareil de traitement d'échantillon (1000) selon la revendication 8, dans lequel l'unité première buse (200) est configurée pour se déplacer sur la première platine (110).

10. Appareil de traitement d'échantillon (1000) selon la revendication 8, dans lequel la première platine (110) est munie d'une unité de mise au rebut de pointes distributrices configurée pour mettre au rebut des pointes distributrices montées sur la pluralité de premières buses (210) et/ou des pointes distributrices montées sur la pluralité de deuxièmes buses (310).

11. Appareil de traitement d'échantillon (1000) selon la revendication 1, dans lequel l'unité de logement de deuxièmes contenants (126) est disposée sur une deuxième platine (120).

12. Appareil de traitement d'échantillon (1000) selon la revendication 11, comprenant un mécanisme de refroidissement configuré pour refroidir l'unité de logement de deuxièmes contenants (126).

13. Appareil de traitement d'échantillon (1000) selon la revendication 11, dans lequel l'unité deuxième buse (300) est configurée pour se déplacer sur la deuxième platine (120).

14. Appareil de traitement d'échantillon (1000) selon la revendication 1, dans lequel la pluralité de porte-contenants (510) logent la pluralité de contenants d'échantillon (514a) à une position plus proche de la pluralité de couloirs de traitement (122) que la pluralité de contenants d'acide nucléique (512a) dans la deuxième direction.

15. Appareil de traitement d'échantillon (1000) selon la revendication 1, dans lequel le mécanisme de conversion d'écartement (500) comporte une pluralité de parties de support de porte-contenant (520), un mécanisme à biellette (530), un élément coulisseau (540), et un mécanisme d'entraînement d'élément coulisseau (550, 560), la pluralité de parties de support de porte-contenant étant configurées pour supporter la pluralité de porte-contenants (510) de sorte que chacun de la pluralité de porte-contenants est mobile dans la première direction, le mécanisme à biellette étant relié à la pluralité de parties de support de porte-contenant, l'élément coulisseau étant configuré pour amener le mécanisme à biellette à coulisser entre l'écartement plus petit et l'écartement plus grand, le mécanisme d'entraînement d'élément coulisseau étant configuré pour entraîner l'élément coulisseau.
